# EUROPÄISCHE PATENTANMELDUNG

(11) **EP 3 367 103 A1**
(43) Veröffentlichungstag der Anmeldung: **29.08.2018**
(21) Anmeldenummer: 17158518.5
(22) Anmeldetag: 28.02.2017
(51) Int. Cl.: G01N 35/00, B25J 15/00, G01N 33/15, G01N 21/17

(54) **VORRICHTUNG UND VERFAHREN ZUR BESTIMMUNG WENIGSTENS EINES PHYSIKALISCHEN PARAMETERS PHARMAZEUTISCHER PRODUKTE**

(71) Anmelder: SOTAX AG, 3600 Thun (CH)
(72) Erfinder: BOSS, Thomas, 3645 Gwatt (CH)
(74) Vertreter: Patentbüro Paul Rosenich AG

(57) **Zusammenfassung**

Eine Vorrichtung zur Bestimmung wenigstens eines physikalischen Parameters pharmazeutischer Produkte, wie beispielsweise Tabletten (T), Pillen, Oblongs oder dergleichen, weist zumindest eine Prüfstation (1) auf. Weiters ist zumindest ein automatisches Transportsystem für die Produkte zu und/oder zwischen und/oder innerhalb und/oder von der oder jeder Prüfstation (1) vorhanden. Das Transportsystem weist zumindest einen Roboterarm (2) mit zumindest einer steuerbaren Halteeinrichtung (3) für die pharmazeutischen Produkte auf. Die Verfahrwege und die räumliche Orientierung der Halteeinrichtung (3) sind frei programmierbar.

## Beschreibung

Die Erfindung betrifft eine Vorrichtung zur Bestimmung wenigstens eines physikalischen Parameters pharmazeutischer Produkte, wie beispielsweise Tabletten, Pillen, Oblongs oder dergleichen, nach dem Oberbegriff des Anspruchs 1, sowie ein Verfahren zur Bestimmung wenigstens eines physikalischen Parameters pharmazeutischer Produkte, wie beispielsweise Tabletten, Pillen, Oblongs oder dergleichen, nach dem Oberbegriff des Anspruchs 16.

Bei der Herstellung tablettenförmiger, pharmazeutischer, chemischer oder lebensmitteltechnischer Produkte, wie etwa Oblongs, Tablets, Tabletten, Pillen oder Dragees, müssen eine Vielzahl von Eigenschaften der Produkte beachtet und eingehalten werden. Zur Kontrolle werden deshalb Prüflinge aus der laufenden Produktion stichprobenartig entnommen, gesammelt und unter anderem in Bezug auf ihre physikalischen Eigenschaften, beispielsweise Gewicht, Härte sowie Zerfallszeit in einem Medium wie auch die Abmessungen des Prüflings untersucht, gemessen und gegebenenfalls registriert.

Die Dokumente US2014145082, US2015310454, US2016109385, GB2524130, US2010328669 und WO2005074553 beschreiben Methoden zur Untersuchung von Tabletten nach vorwiegend lichtoptischer Art. Die DE102008035830 offenbart eine Vermessung von Tabletten, sowie einen Härtetest.

Durch die WO0190744 ist eine Vorrichtung zur automatischen Qualitätskontrolle von tablettenförmigen Prüflingen bekannt geworden. Die Vorrichtung besteht aus einer Zuführeinrichtung, welche die gesammelten Prüflinge vereinzelt, und einem Transportstern mit peripher angeordneten Aufnahmekammern für je einen Prüfling, in welche die Prüflinge zugeführt werden. In den Aufnahmekammern abgelegte Prüflinge werden mittels des Transportsterns schrittweise einer oder mehreren Prüfstationen zugeführt und, sofern es sich um zerstörungsfreie Prüfungen handelt, anschließend beispielsweise in einen Linearförderer ausgeworfen und zu Archivierungszwecken gesammelt. Bei einer zerstörenden Prüfung der Prüflinge findet der Auswurf der zerstörten Prüflinge meist direkt in der jeweiligen Prüfstation statt, beispielsweise durch eine Öffnung in einer die Aufnahmekammern nach unten begrenzenden Auflage, auf welcher der Transportstern dreht. Bei den Prüfstationen handelt es sich beispielsweise um einen Härtetester, eine Waage sowie einer Einrichtung zur Bestimmung der Abmessungen, wie etwa Länge und/oder Breite und/oder Dicke der Prüflinge.

Zur Bestimmung der Wirkstoffe der Prüflinge ist bekannt, eine berührungsfreie Messung durchzuführen, bei der die Prüflinge einzeln von elektromagnetischen Wellen geeigneter Wellenlänge durchleuchtet werden. Als besonders zweckmäßig hat sich hierbei die so genannte NIR-Prüfung (NIR: Near Infra Red) erwiesen, bei der elektromagnetische Wellen des nahen Infrarotbereichs zur Durchleuchtung der Prüflinge verwendet werden.

Beispielsweise ist ein System bekannt, das aus einem automatischen Tablettenprüfgerät (für z.B. Gewicht, Dicke, Durchmesser und Härte) und einem NIR-Prüfsystem besteht. Die Tablette wird nach der Messung von Gewicht und der Dicke ausgeschleust und in einen Tablettenhalter hineinvibriert. Dieser Tabletten-Halter wird zum NIR-System gebracht, die Messung gestartet und danach wird die Tablette in ein Röhrchen oder Magazin abgelegt.

Nachteilig an diesem Verfahren ist beispielsweise, dass die Tablettennester für die unterschiedlichen Tablettenformen teuer und auch sehr heikel in der Herstellung sind. Damit sich die Tabletten hineinvibrieren lassen, wird der Halter leicht grösser als die eigentliche Tablette hergestellt. Dies führt dazu, dass eventuell unerwünschtes Streulicht (Licht das nicht durch die Tablette durchgegangen ist) das Messresultat verfälschen kann.

Nach dem Pressvorgang sind die Tabletten teilweise noch warm und deshalb leicht grösser als im abgekühlten Zustand. Die Detektion (ist die Tablette wirklich im Halter und zu 100% orientiert) ist aufwändig und nicht prozesssicher. Nach der Messung z.B. mit NIR werden die Tabletten individuell z.B. für nachfolgende Kontrollmessungen in einem Tabletten-Magazin abgelegt. Dazu wird die Tablette mit einem Luftstrom, aus dem Halter hinausgeblasen und über eine Orientierungsrinne in das entsprechende Röhrchen abgelegt. Die dazu benötigten Teile sind tablettenspezifisch hergestellt. Nachteilig ist auch hier die Prozesssicherheit (Überwachung mit Sensoren sehr aufwendig)

Im Patent EP2160599 ist die Positionierung und Orientierung eines Objekts über eine Auflagefläche eines Schiebers beschrieben. Nachteilig ist hier, dass die Unterseite des Schiebers z.B. bei konkaven Tablette tablettenspezifisch angepasst werden muss. Auch die Verhinderung des unerwünschten Streulichts ist nicht zu 100% gelöst. Der hier eingesetzte Schieber kann ein Nachteil sein, wenn man die Tablette an genau definierte Positionen bringen oder dort aufnehmen will oder direkt nach der Messung beispielsweise in ein Röhrchen versorgen will.

Um ein verifizierbares Maß für die Qualitätskontrolle zu erhalten, ist es bei nicht scheibenförmigen, sondern beispielsweise tropfen-, oliven-, zylinder- oder torpedoförmigen Prüflingen sowie bei Prüflingen mit bombierten Formen erforderlich, dass die Durchleuchtung immer an derselben Stelle bzw. entlang der selben Achse der Prüflinge stattfindet.

Aufgabe der vorliegenden Erfindung ist es somit, eine Vorrichtung zu schaffen und ein Verfahren anzugeben, welche die vorgenannten Nachteile nicht aufweisen und insbesondere mit geringem Aufwand eine exakte Positionierung und gegebenenfalls auch Ausrichtung eines Objekts, z. B. eines mittels einer Testapparatur zu prüfenden Pharmazeutikums, auf einer Unterlage bzw. Auflagefläche ermöglichen.

Die Aufgabe wird durch die Merkmale der unabhängigen Ansprüche 1 und 16 gelöst. Vorteilhafte Weiterbildungen sind in den Figuren und in den abhängigen Patentansprüchen dargelegt.

Ausgangspunkt ist eine Vorrichtung zur Bestimmung wenigstens eines physikalischen und/oder chemischen Parameters pharmazeutischer Produkte, mit zumindest einer Prüfstation sowie mit zumindest einem automatischen Transportsystem für die Produkte zu und/oder zwischen und/oder innerhalb und/oder von der oder jeder Prüfstation. Dabei kann es sich bei den Produkten um beispielsweise Tabletten, Pillen, Oblongs oder dergleichen handeln.

Gemäss der Erfindung ist eine derartige Vorrichtung dadurch gekennzeichnet, dass das Transportsystem eine steuerbare Halteeinrichtung zur kraft- und/oder formschlüssigen Aufnahme der pharmazeutischen Produkte aufweist, wobei die Verfahrwege und die räumliche Orientierung der Halteeinrichtung frei programmierbar sind.

Damit ist eine genau definierte Verbindung des Produktes mit der Transporteinrichtung gewährleistet, durch welche in der anschliessend vom Produkt durchlaufenen Testprozedur jederzeit die genaue Position und räumliche Lage des Produkts bekannt ist und eingestellt werden kann. Die Handhabung ist damit besonders flexibel möglich und Fehlmessungen durch Positionierungsfehler können minimiert werden.

Gemäss einer bevorzugten Ausführungsform ist die Halteeinrichtung an einem vorzugsweise frei programmierbaren Roboterarm angeordnet. Diese bewährte Technik gestattet in einfacher Weise das Anfahren jeder beliebigen Position mit dem pharmazeutischen Produkt und dessen Ausrichtung in jede gewünschte räumliche Lage.

Bevorzugt ist erfindungsgemäss vorgesehen, dass zumindest eine der Prüfstationen eine Einrichtung für eine Durchstrahlungs- oder Reflexionsmessung ist. Vorzugsweise umfasst diese Einrichtung eine Auf- oder Anlagefläche für das pharmzeutische Produkt.

Eine vorteilhafte Ausführungsform der erfindungsgemässen Vorrichtung ist dadurch gekennzeichnet, dass die Einrichtung zur Durchstrahlungs- oder Reflexionsmessung eine für die verwendeten Wellenlängen undurchlässige und zumindest während der Durchstrahlungsmessung das Produkt zum Teil formschlüssig aufnehmende Auflage aufweist, in welcher eine durch das Produkt vollständig abdeckbare Durchlassöffnung ausgearbeitet ist. Damit lassen sich Fehlmessungen durch Fremd- und Streulicht vermindern.

Um die Verfälschung der Messung durch Streulicht noch weiter zu vermindern, besteht vorteilhafterweise die Auflage aus einem elastisch nachgiebigem Material. In dieses Material kann das pharmazeutischen Produkt mittels des Transportsystems formschlüssig hineingedrückt werden, da das Transportsystem aufgrund der form- bzw. kraftschlüssigen Aufnahme des Produktes entsprechende Kräfte auf das Produkt ausüben kann.

Wenn bevorzugt die Halteeinrichtung des Roboterarms zumindest zwei relativ zueinander verstellbare Greiferbacken aufweist, kann die erfindungsgemässe Vorrichtung einfach und rasch auf unterschiedliche pharmazeutische Produkte eingestellt werden, die Ansteuerung zur Aufnahme des Produktes ist sehr einfach und das Produkt kann sehr flexibel aufgenommen, transportiert, positioniert und abgelegt werden.

Eine vorteilhafte Ausführungsform sieht dafür vor, dass die der jeweils gegenüberliegenden Greiferbacke zugewandte Greiffläche derart geformt ist, dass für das Produkt zwischen den Greiferbacken eine zumindest Zweipunkt-Auflage gebildet ist. Damit ist in einfacher Weise eine gute kraftschlüssige Aufnahme des Produktes durch das Transportsystem zu bewerkstelligen.

Eine besonders gute Haltewirkung für den sicheren Transport der pharmazeutischen Produkte kann sichergestellt werden durch eine alternative Ausführungsform, bei welcher die Greifflächen der Greiferbacken komplementär zu zumindest einem Teil der Umfangskontur der Produkte geformt sind.

Eine vorteilhafte weil konstruktiv einfache und in geringer Baugrösse ausführbare Ausführungsform der Erfindung ist dadurch gekennzeichnet, dass die Greiferbacken einander zangenförmig gegenüberliegen und aufeinander zu schliessbar und voneinander weg offenbar sind.

Alternativ ist auch eine Ausführungsform möglich, bei welcher die Greiferbacken entlang einer geschlossenen Kontur angeordnet sind und einen zentralen Freiraum zur Aufnahme des pharmazeutischen Produktes aufweisen. Diese Variante bietet den Vorteil der bestmöglichen Aufnahme und Haltewirkung durch das Transportsystem auf das pharmazeutischen Produkt.

Besonders vorteilhaft ist bei allen Greifervarianten und Ausführungsformen des Transportsystems, wenn die steuerbare Halteeinrichtung und/oder allfällige Greiferbacken Sensorik zur Ermittlung des Weges allfälliger Greiferbacken und/oder der auf die pharmazeutischen Produkte ausgeübten Kraft aufweist. Durch eine derartige Lösung kann bereits beim Aufnehmen des Produktes durch das Transportsystem eine Prüfung auf die räumliche Lage und Position des Produktes durchgeführt werden, indem die ermittelten Werte für Kraft und/oder Weg beispielsweise der Greiferbacken mit Referenzwerten für die korrekte Lage verglichen werden. Dies verhindert in weiterer Folge Fehlmessungen aufgrund fehlerhafter Positionierung des Produktes.

Eine weitere vorteilhafte Ausführungsform der erfindungsgemässen Vorrichtung sieht vor, dass an der Übergabeposition des pharmazeutischen Produktes an das Transportsystem ein Drehteller angeordnet ist, auf welchem das Produkt unmittelbar vor der kraft- und/oder formschlüssigen Aufnahme durch das Transportsystem zu liegen kommt. So kann bei Feststellung eines falsch orientierten pharmazeutischen Produktes (z.B. tropfenförmige Tablette falsch orientiert für komplementär geformte Greifer), sei es über eine Greifersensorik, über optische Lageerkennungssysteme od. dgl., das Produkt mittels des Drehtellers in die korrekte Lage für die Übernahme durch das Transportsystem gedreht werden.

Die Haltewirkung kann bei gleichzeitiger Schonung der Produkte vor Beschädigung bei der Handhabung noch verbessert werden, wenn gemäss einem weiteren optionalen Merkmal der Erfindung die Greiffläche zumindest einer der Greiferbacken mit einer elastischen Schicht bedeckt ist. Ergänzend oder alternativ dazu kann die die Greiferbacken auskleidende Schicht aus einem für die bei der Durchstrahlungsmessung verwendeten Wellenlängen undurchlässigen Material bestehen, um damit Fehlmessungen durch Streu- als auch Fremdlicht weitestgehend zu verhindern. Diese Lösung ist auch für Durchstrahlungs- oder Reflexionsmessungen mit Tablettenauflagen ohne elastische Deckschickt besonders vorteilhaft.

Eine vorteilhafte Ausführungsform der erfindungsgemässen Vorrichtung ist dadurch gekennzeichnet, dass die Greiferbacken nach innen hin über ein geklemmtes Produkt ragende Auskragungen aufweisen, und dass die den Boden der Auskragung überragende Höhe der Greiferbacken geringer ist als die Dicke der Produkte. Damit können die Haltewirkung als auch die Verhinderung von Streulicht bei Durchlicht- bzw. Durchstrahlungsmessungen noch weiter verbessert werden.

Besonders bevorzugt ist eine Erfindungsvariante, bei welcher das Transportsystem zur Ausübung einer auf das Produkt in Richtung auf die Auflage hin wirkenden Anpresskraft ausgelegt ist.

Die Lösung der eingangs gestellten Aufgabe ist auch für ein Verfahren zur Bestimmung wenigstens eines physikalischen Parameters pharmazeutischer Produkte, wie beispielsweise Tabletten, Pillen, Oblongs oder dergleichen, möglich. Dabei wird zumindest eine chemische oder physikalische Eigenschaft geprüft und das Produkt zwischen und/oder während und/oder nach zumindest einem Prüfvorgang durch ein automatisches Transportsystem transportiert und gehandhabt.

Erfindungsgemäss ist zur Lösung der Aufgabe dabei vorgesehen, dass der Transportweg des Produktes und dessen räumliche Orientierung zumindest zum Teil unabhängig von der räumlichen apparativen Anordnung vorprogrammiert und vorzugsweise mittels eines Roboterarms abgefahren wird.

Bevorzugt wird zusätzlich zumindest eine Eigenschaft des Produkts mittels einer Durchstrahlungs- oder Reflexionsmessung ermittelt.

Gemäss einer vorteilhaften Verfahrensvariante ist vorgesehen, dass zumindest für die Durchstrahlungsmessung das Produkt in Bezug auf die Einrichtung zur Durchstrahlungsmessung ortsfest fixiert wird, vorzugsweise in Richtung einer Auflage mit einer Andruckkraft beaufschlagt wird.

Alternativ oder auch ergänzend dazu kann auch vorgesehen sein, dass das Produkt während der Durchstrahlungs- oder Reflexionsmessung beweglich belassen wird. Allenfalls können auch mehrere Messungen durchgeführt werden, vorzugsweise an unterschiedlichen Stellen des Produktes. So könnte bei länglichen Produkten wie beispielsweise Oblongs eine Durchstrahlung oder auch eine Reflexionsmessung in der Mitte und in den beiden Endabschnitten stattfinden, um beispielsweise einen Mittelwert zu bestimmen..

Vorteilhafterweise wird das Produkt während zumindest eines Teils des Transportweges mit dem Transportsystem, vorzugsweise einer Halteeinrichtung des Transportsystems, kraftschlüssig und/oder formschlüssig gekoppelt.

Eine weitere erfindungsgemässe Verfahrensvariante ist dadurch gekennzeichnet, dass das Produkt zumindest nach Abschluss aller Prüfungen mittels des Transportsystems in einer Ablage abgelegt oder an weitere Prüfsysteme, Ablagen oder Handhabungssysteme übergeben wird.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung beschrieben sind.

Die Bezugszeichenliste ist wie auch der technische Inhalt der Patentansprüche und Figuren Bestandteil der Offenbarung. Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Es zeigen dabei:
- Fig. 1: einen Abschnitt einer ersten Ausführungsform einer erfindungsgemässen Vorrichtung,
- Fig. 2: eine perspektivische Ansicht einer Haltevorrichtung in erfindungsgemässer Ausführung,
- Fig. 3: ein Längsschnitt durch eine Haltevorrichtung gemäss Fig. 2, aufgelegt auf ein Tablettennest einer Durchstrahlungsmessung,
- Fig. 4: eine perspektivische Ansicht einer alternativen Ausführung einer Haltevorrichtung,
- Fig. 5: einen weiteren Ausschnitt einer Ausführungsform einer erfindungsgemässen Vorrichtung in perspektivischer Ansicht,
- Fig. 6: eine schematische Darstellung einer Greiferbackenanordnung mit geschlossener Kontur, und
- Fig. 7: ebenfalls eine schematische Darstellung einer weiteren Greiferbackenanordnung mit geschlossener Kontur.

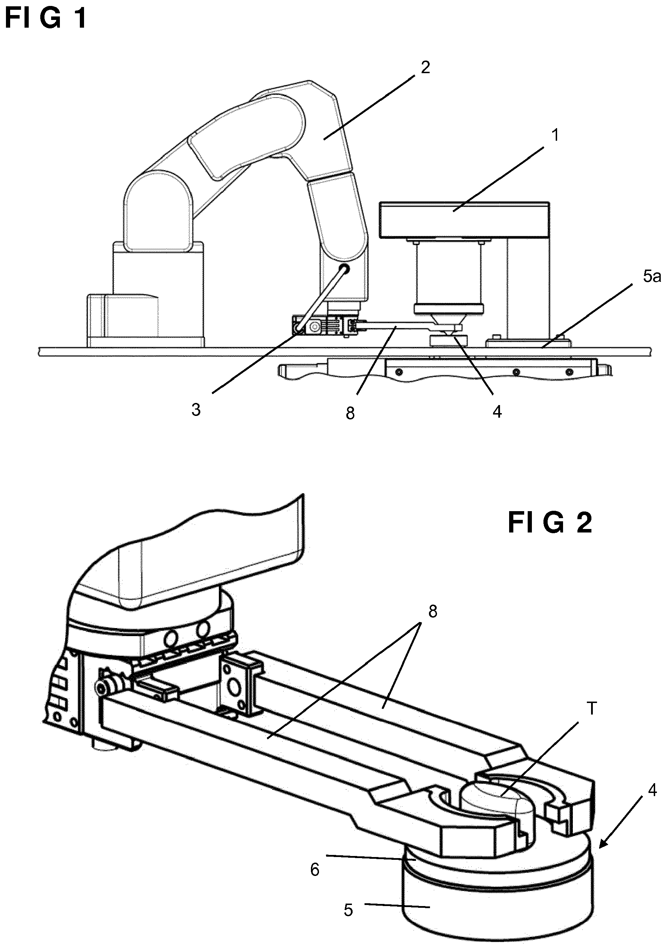

Fig. 1 zeigt einen Ausschnitt einer Vorrichtung zur Bestimmung wenigstens eines physikalischen Parameters pharmazeutischer Produkte. Dies können Tabletten, Pillen, Oblongs oder dergleichen sein. Die Vorrichtung weist zumindest eine Prüfstation 1 sowie zumindest ein automatisches Transportsystem für die Produkte zu und/oder zwischen und/oder innerhalb und/oder von der oder jeder Prüfstation 1 auf. Dabei erfolgt die Bestimmung wenigstens eines physikalischen Parameters dieser pharmazeutischen Produkte. Zumindest eine chemische oder physikalische Eigenschaft werden geprüft, wobei das Produkt zwischen und/oder während und/oder nach dem zumindest einem Prüfvorgang durch ein automatisches Transportsystem transportiert und gehandhabt wird.

Das Transportsystem umfasst erfindungsgemäss zumindest einen Roboterarm 2 mit zumindest einer steuerbaren Halteeinrichtung 3 für die pharmazeutischen Produkte. Vorzugsweise sind die Verfahrwege des Roboterarms 2 selbst und auch jene der Halteeinrichtung 3 sowie die räumliche Orientierung der Halteeinrichtung 3 an diesem Roboterarm 2 frei programmierbar. Dam it kann auch das pharmazeutische Produkt räumlich an jede beliebige Position innerhalb der Reichweite des Roboterarms 2 gefahren werden, und das bei entsprechend beweglicher Anbringung der Halteeinrichtung 3 am freien Ende des Roboterarms 2 auch noch in jeder beliebigen räumlichen Orientierung. Insbesondere kann der Roboterarm 2 derart angesteuert werden, dass der Transportweg des Produktes und dessen räumliche Orientierung zumindest zum Teil unabhängig von der räumlichen apparativen Anordnung vorprogrammiert und mittels des Roboterarms 2 abgefahren wird.

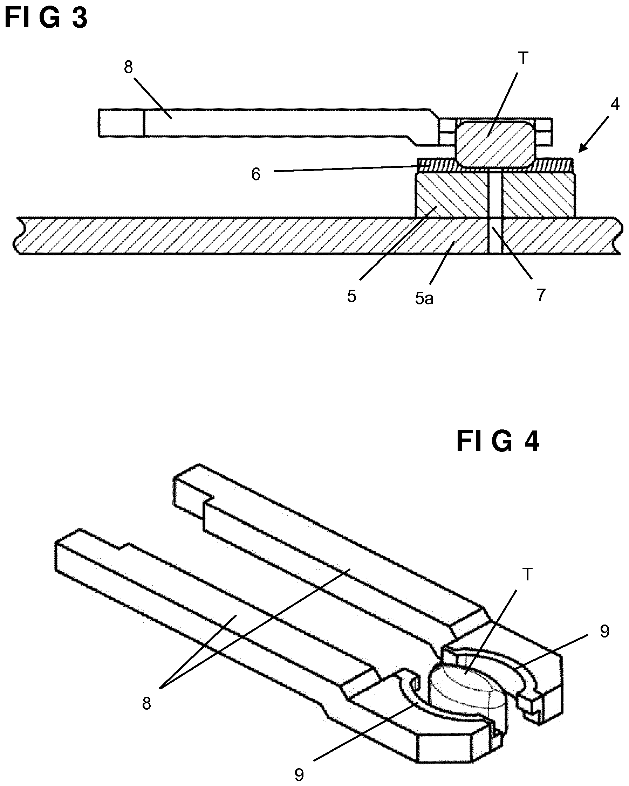

Zumindest eines der Prüfstationen 1 ist eine Einrichtung für eine Durchstrahlungs- oder Reflexionsmessung. So sind zur Bestimmung des aktiven Wirkstoffs in einem pharmazeutischen Produkt Messungen mit NIR(Near Infra Red)- , RAMAN- oder ähnlichen Detektoren üblich. Insbesondere für die Durchführung derartiger Durchstrahlungs- oder Reflexionsmessungen im Rahmen eines Prüfzyklus für pharmazeutische Produkte wird dieses Produkt, beispielsweise die in den Fig. 2 bis 4 dargestellte Tablette T, mittels des Roboterarms 2 zu einem Tablettennest 4 (siehe Fig. 2 und 3) verbracht. Dieses besteht vorzugsweise aus einem festen, meist metallischen Unterteil 5, allenfalls Teil einer Trägerstruktur 5a, und einer darauf befestigten, Aufnahme aus elastisch nachgiebigem Material, insbesondere einer Kunststoffscheibe 6. Durch das Zentrum des Unterteils 5 und der Kunststoffscheibe 6 führt eine Bohrung, allenfalls durch ein für die eingesetzte Strahlung durchlässiges Fenster geschützt, welche Bohrung eine durch das Produkt vollständig abdeckbare Durchlassöffnung für die Strahlung zur Tablette T darstellt. Die für die Messung genutzte Strahlung kann beispielsweise mittels eines Strahlungsleiters 7 zugeführt werden. Das Unterteil 5 und die Kunststoffscheibe 6 hingegen sind für die Strahlung undurchlässig. Auch der Roboterarm 2 kann auf der Trägerstruktur 5a befestigt sein.

Für alle Vorgänge der Handhabung der pharmazeutischen Produkte ist die nachfolgend beschriebene Weise des Greifens und Klemmens der Produkte vorteilhaft. Ganz besonders gilt dies aber für die Handhabungsvorgänge in Bezug auf die Durchstrahlungs- oder Reflexionsmessung.

Die Tablette T wird vorteilhafterweise mittels zweier relativ zueinander verstellbarer Greiferbacken 8 der Halteeinrichtung 3 am freien Ende des Roboterarms 2 vorzugsweise formschlüssig geklemmt und damit sicher gehalten. Die Greiferbacken 8 können gemäss einer vorteilhaften Ausführungsform auch federnd ausgelegt sein, um die Grössen-Toleranzen der Produkte auszugleichen oder auf die Grössenveränderung im Zuge einer Temperaturänderung der Produkte (Veränderung bei der Tablettenherstellung von warm zu kalt) zu reagieren. Aufgrund dieser sicheren Klemmung kann das pharmazeutische Produkt durch die Halteeinrichtung 3 des Roboterarms 2 nicht nur abgelegt, sondern mit einer einstellbaren Kraft auf die Kunststoffscheibe 6 gedrückt werden, so dass sich ein Formschluss zwischen der Unterseite der Tablette T und der Oberseite der Kunststoffscheibe 6 ergibt, der jeglichen Spalt für unerwünschtes Streulicht bzw. Streustrahlung zum Strahlungsdetektor verhindert. Natürlich ist dazu das Transportsystem, insbesondere der Roboterarm 2, zur Ausübung einer auf das Produkt in Richtung auf die Auflage hin wirkenden Anpresskraft ausgelegt. Damit wird zumindest für die Durchstrahlungs- oder Reflexionsmessung das Produkt T in Bezug auf die Einrichtung 1 ortsfest fixiert. Diese Fixierung wird vorzugsweise erzielt oder unterstützt dadurch, dass das Produkt in Richtung der Auflage mit einer Andruckkraft beaufschlagt wird. Jedenfalls wird gemäss der vorliegenden Erfindung das Produkt während zumindest eines Teils des Transportweges mit dem Transportsystem, vorzugsweise einer Halteeinrichtung 3 des als Roboterarm 2 gestalteten Transportsystems, kraftschlüssig und/oder formschlüssig mit dem Transportsystem gekoppelt.

Die Fixierung der pharmazeutischen Produkte für die Durchstrahlungs- oder Reflexionsmessung ist aber nicht zwingend. Vielmehr kann es für bestimmte Messungen vorteilhaft sein, wenn das Produkt während der Durchstrahlungs- oder Reflexionsmessung beweglich belassen wird. Eine beispielsweise in Greiferbacken 8 geklemmte Tablette T wird durch Öffnen der Greiferbacken 8 nach dem Ablegen des Produktes kurzzeitig und nur für die Durchstrahlung- oder Reflexionsmessung freigegeben. Nach Abschluss dieses Prüfschrittes wird das Produkt T vorzugsweise wieder in gleicher Weise wie oben erläutert durch Ergreifen mit der Halteeinrichtung 3 des Transportsystems mit diesem System gekoppelt. Speziell am beweglich belassenen Produkt T, allenfalls aber auch an mit dem Transportsystem gekoppelt belassenen Produkten, können vorteilhafterweise mehrere Messungen durchgeführt werden, vorzugsweise an unterschiedlichen Stellen des Produktes T. Insbesondere können bei länglichen oder grossflächigen Produkten, wie beispielsweise Oblongs, Messungen an unterschiedlichen Stellen des Produktes durchgeführt und ein Mittelwert für den Messwert über das gesamte Produkt bestimmt werden.

Um die Haltewirkung der Halteeinrichtung 3 am Roboterarm 2 auf die pharmazeutischen Produkte noch zu verbessern, flexibler zu gestalten und teilweise auch die Streustrahlung noch sicherer vermeiden zu können, sind insbesondere folgende Varianten der Greiferbacken 8 im Rahmen der Erfindung möglich.

So kann die der jeweils gegenüberliegenden Greiferbacke 8 zugewandte Greiffläche derart geformt sein, dass für das Produkt zwischen den Greiferbacken 8 eine zumindest Zweipunkt-Auflage gebildet ist. Andererseits könnten die Greifflächen der Greiferbacken 8 auch komplementär zu zumindest einem Teil der Umfangskontur der Produkte geformt sein. Für eine sehr schonende Klemmung der Produkte in den Greiferbacken 8 kann eine Ausführungsform vorteilhaft sein, bei welcher die Greiffläche zumindest einer der Greiferbacken 8 mit einer elastischen Schicht 9 vorzugsweise aus Kunststoffmaterial bedeckt ist. Diese Schicht 9 kann auch eine zusätzliche Abdichtung zur Vermeidung von unerwünschter Streustrahlung bei der Durchstrahlungsmessung darstellen.

Jedenfalls ist die Halteeinrichtung 3 derart ausgelegt, dass wenigstens eine der Greiferbacken 8 zumindest eine Aussparung aufweist, welche so geformt ist, dass sich zwischen den Kontaktflächen ein Zwischenraum zur Aufnahme des pharmazeutischen Produktes befindet, welcher zum Öffnen der Anordnung der Greiferbacken 8 vergrösserbar und zu zum Schließen verkleinerbar ist. Die Greiferbacken 8 sind - gegebenenfalls adaptiert oder adaptierbar an das Produkt - so geformt, dass das zu prüfende Produkt bei geöffneten Greiferbacken 8 über eine Zugangsöffnung in den Zwischenraum einbringbar und in diesem bei geschlossener Anordnung der Greiferbacken 8 formschlüssig und/oder kraftschlüssig im Zwischenraum zwischen den Greiferbacken 8 gehalten ist. Dazu wird vorzugsweise wie folgt vorgegangen: Die Greiferbacken 8 der Halteeinrichtung 3 werden geöffnet, danach wird das Produkt auf eine Auflagefläche aufgebracht, die Halteeinrichtung 3 wird vorzugsweise so bewegt, dass das Objekt in dem Zwischenraum zu liegen kommt. Schliesslich werden die Greiferbacken 8 geschlossen und hierdurch wird das Produkt an einer durch die Form der Kontaktflächen festgelegten Stelle auf der Auflagefläche positioniert und ggf. ausgerichtet. Bevorzugt werden alle Schritte vollautomatisch, z. B. rechnergesteuert, durchgeführt.

Weiters könnten speziell für die Ausübung einer auf das Tablettennest 4 hin gerichteten Anpresskraft auf die Tablette T oder jedes andere pharmazeutische Produkt an den Greiferbacken 8 nach innen hin über ein geklemmtes Produkt T ragende Auskragungen vorgesehen sein, die also die freie Öffnung oberhalb des Produktes etwas einengen. Die den Boden der Auskragung überragende Höhe der Greiferbacken 8 ist dabei vorzugsweise geringer ist als die Dicke der Produkte.

Die bislang beispielhafte erläuterte Variante der Erfindung verwendete einander zangenförmig gegenüberliegende und aufeinander zu schliessbare und voneinander weg öffenbare Greiferbacken 8. Diese sind vorzugsweise parallel zueinander angeordnet und im Wesentlichen quer zu deren Längserstreckung bewegbar, vorzugsweise durch eine Basiseinheit der Halteeinrichtung 3 gehalten und mittels dieser Halteeinrichtung 3 mit beispielsweise dem Roboterarm 2 verbunden. Besonders vorteilhaft sind die Greiferbacken 8 austauschbar in der Halteeinrichtung 3 befestigt, um sie an verschiedene Arten von pharmazeutischen Produkten T anzupassen oder im Falle von Störungen zu reparieren oder zu ersetzen.

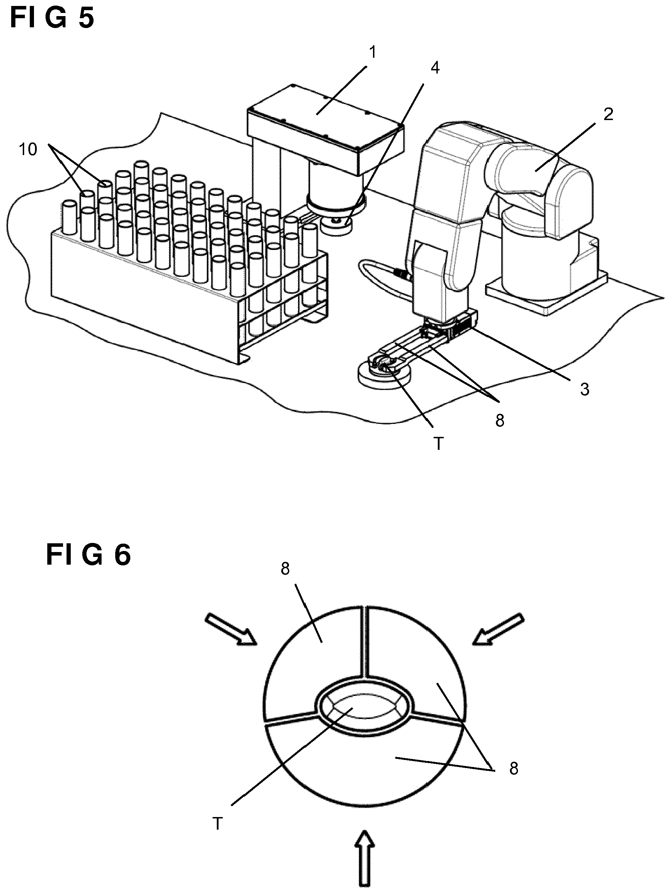

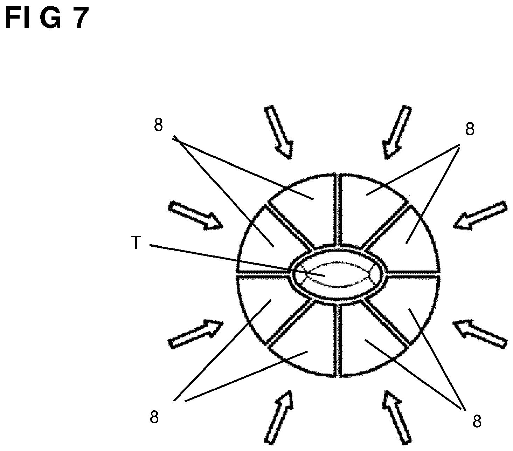

Alternativ ist neben derartigen Zangengreifern, die allenfalls durch Ausstosser für allenfalls an den Greiferbacken 8 festklebenden Produkten T ergänzt werden können, auch Ausführungsformen möglich, bei welcher die Greiferbacken 8 entlang einer geschlossenen Kontur angeordnet. In den Fig. 6 und 7 sind derartige Anordnungen von Greiferbacken 8 dargestellt, die das pharmazeutische Produkt T vorzugsweise komplett entlang von dessen Umfang umgeben und klemmen können. Sie weisen einen zentralen Freiraum zur Aufnahme des pharmazeutischen Produktes T auf und gewährleisten eine besonders gute Aufnahme und Haltewirkung auf das pharmazeutische Produkt T. Vorzugsweise sind die dem Produkt T zugewandten Konturen der Greiferbacken 8 genau komplementär zur äusseren Umfangskontur des Produktes T ausgebildet.

Bei allen Greifervarianten und Ausführungsformen von Greiferbacken 8 und Transportsystem kann die steuerbare Halteeinrichtung und/oder zumindest eine der allfälligen Greiferbacken 8 eine Sensorik zur Ermittlung des Weges allfälliger Greiferbacken 8 und/oder der auf die pharmazeutischen Produkte T ausgeübten Kraft aufweisen. Über einen Wegsensor kann der während der Aufnahme des Produktes T durchfahrene Arbeitsweg der jeweiligen Aufnahmestruktur, beispielsweise zumindest einer Greiferbacke 8, ermittelt werden. Dieser aktuelle Weg wird in der Steuerung der Halteeinrichtung 3 oder des gesamten Transportsystems mit einem Referenzwert verglichen, der für einen korrekten Greifvorgang bei korrekter Ausrichtung des pharmazeutischen Produktes T vorab ermittelt wurde. So kann eine Unterschreitung des Referenzwertes auf eine fehlerhafte Ausrichtung des Produktes T hinweisen, eine Überschreitung hingegen auf eine unbeabsichtigte Zerstörung des Produktes beim Greifvorgang oder auch darauf, dass gar kein Produkt zur Übergabe an das Transportsystem 2, 3 vorhanden ist.

In ähnlicher Weise kann über eine Bestimmung der zum Ergreifen des Produktes erforderlichen Kraft und dem Vergleich dieses Wertes mit einem Referenzwert auf das Vorhandensein und/oder die korrekte Ausrichtung des Produktes rückgeschlossen werden. Eine grössere Kraft als der Referenzwert lässt auf entweder eine fehlerhafte Greiferfunktion mit erhöhter Reibung oder Klemmen der Greiferbacken 8 schliessen, oder weist auf eine nicht korrekte Ausrichtung des Produktes T hin. Kraftmesswerte unterhalb des Referenzwertes hingegen können durch ein nicht vorhandenes Produkt oder ein falsches Produkt (mit geringerer Festigkeit als erwartet) entstehen.

Beide Vorgangsweisen einzeln oder auch in Kombination können natürlich in weiterer Folge Fehlmessungen aufgrund fehlerhafter Positionierung des Produktes bzw. durch ein falsches Produkt verm eiden.

Sollte eine falsche Ausrichtung des Produktes T ermittelt worden sein, kann eine weitere Ausführungsform der erfindungsgemässen Vorrichtung mit einem Drehteller an der Übergabeposition des pharmazeutischen Produktes an das Transportsystem zum Einsatz kommen. Auf einem derartigen Drehteller kann das Produkt T unmittelbar vor der kraft- und/oder formschlüssigen Aufnahme durch das Transportsystem bei Feststellung einer falschen Ausrichtung in die korrekte Lage für die Übernahme durch das Transportsystem gedreht werden.

Aufgrund der freien Programmierbarkeit des Roboterarms 2 und der Tatsache, dass keinerlei vorgegebene Wege für die pharmazeutischen Produkte aufgrund von Beschränkungen oder Wegvorgaben des Transportsystems vorliegen, können die Prüfstationen der erfindungsgemässen Vorrichtung in jeder beliebigen Reihenfolge angefahren werden. So besteht beispielsweise die Möglichkeit, nach Durchstrahlungs- oder Reflexionsmessungen das pharmazeutische Produkt wieder in eine Prüfstation 1 oder ein komplettes Tablettenprüfgerät zurücklegen, um z.B. die Härte oder andere physikalische Parameter zu messen.

Andererseits könnten die Tabletten T oder andere Produkte nach der Durchstrahlungs- oder Reflexionsmessung auch direkt in ein Röhrchen 10 oder ähnliches Magazin versorgt werden, mit frei programmierten Absetzpositionen, wie in Fig.5 Nach Anfahren dieser Positionen durch den Roboterarm, vorzugsweise mit der Mitte des Produktes T innerhalb des Randes des Röhrchens 10, fällt nach dem Öffnen der Greiferbacken 8 der Halteeinrichtung 3 das Produkt automatisch ins Röhrchen 10. Das Produkt kann aber auch zumindest nach Abschluss aller erwünschten Prüfungen mittels des Transportsystems an weitere Prüfsysteme oder Handhabungssysteme übergeben werden.

### Bezugszeichenliste

- 1: Prüfstation
- 2: Roboterarm
- 3: Halteeinrichtung
- 4: Tablettennest
- 5: Unterteil
- 5a: Trägerstruktur
- 6: Kunststoffscheibe
- 7: Strahlungsleiter
- 8: Greiferbacken
- 9: Elastische Schicht
- 10: Tablettenröhrchen
- T: Tablette

## Patentansprüche

1. Vorrichtung zur Bestimmung wenigstens eines physikalischen und/oder chemischen Parameters pharmazeutischer Produkte (T), wie beispielsweise Tabletten, Pillen, Oblongs oder dergleichen, mit zumindest einer Prüfstation (1) sowie mit zumindest einem automatischen Transportsystem für die Produkte zu und/oder zwischen und/oder innerhalb und/oder von der oder jeder Prüfstation, **dadurch gekennzeichnet, dass** das Transportsystem eine steuerbare Halteeinrichtung (3) zur kraft- und/oder formschlüssigen Aufnahme der pharmazeutischen Produkte (T) aufweist, wobei die Verfahrwege und die räumliche Orientierung der Halteeinrichtung (3) frei programmierbar sind.

2. Vorrichtung nach Anspruch 1, **dadurch gekennzeichnet, dass** die Halteeinrichtung (3) an einem vorzugsweise frei programmierbaren Roboterarm (2) angeordnet ist.

3. Vorrichtung nach Anspruch 1 oder 2, **dadurch gekennzeichnet, dass** zumindest eine der Prüfstationen (1) eine Einrichtung für eine Durchstrahlungs- oder Reflexionsmessung ist, die eine Auf- oder Anlagefläche (5) für das pharmzeutische Produkt (T) aufweist.

4. Vorrichtung nach Anspruch 3, **dadurch gekennzeichnet, dass** die Einrichtung zur Durchstrahlungsmessung eine für die verwendeten Wellenlängen undurchlässige und zumindest während der Durchstrahlungsmessung das Produkt zum Teil formschlüssig aufnehmende Auflage (6) aufweist, in welcher eine durch das Produkt (T) vollständig abdeckbare Durchlassöffnung ausgearbeitet ist.

5. Vorrichtung nach Anspruch 3 oder 4, **dadurch gekennzeichnet, dass** die Auflage (6) aus einem elastisch nachgiebigem Material besteht.

6. Vorrichtung nach einem der Ansprüche 1 bis 5, **dadurch gekennzeichnet, dass** die Halteeinrichtung (3) zumindest zwei relativ zueinander verstellbare Greiferbacken (8) aufweist.

7. Vorrichtung nach Anspruch 6, **dadurch gekennzeichnet, dass** die der jeweils gegenüberliegenden Greiferbacke (8) zugewandte Greiffläche derart geformt ist, dass für das Produkt zwischen den Greiferbacken (8) eine zumindest Zweipunkt-Auflage gebildet ist.

8. Vorrichtung nach Anspruch 6, **dadurch gekennzeichnet, dass** die Greifflächen der Greiferbacken (8) komplementär zu zumindest einem Teil der Umfangskontur der Produkte (T) geformt sind.

9. Vorrichtung nach einem der Ansprüche 6 bis 8, **dadurch gekennzeichnet, dass** die Greiferbacken (8) einander zangenförmig gegenüberliegen und aufeinander zu schliessbar und voneinander weg öffenbar sind.

10. Vorrichtung nach einem der Ansprüche 6 bis 8, **dadurch gekennzeichnet, dass** die Greiferbacken entlang einer geschlossenen Kontur angeordnet sind und einen zentralen Freiraum zur Aufnahme des pharmazeutischen Produktes (T) aufweisen.

11. Vorrichtung nach einem der Ansprüche 1 bis 10, **dadurch gekennzeichnet, dass** die steuerbare Halteeinrichtung (3) und/oder allfällige Greiferbacken (8) Sensorik zur Ermittlung des Weges allfälliger Greiferbacken (8) und/oder der auf die pharmazeutischen Produkte (T) ausgeübten Kraft aufweist.

12. Vorrichtung nach einem der Ansprüche 1 bis 11, **dadurch gekennzeichnet, dass** an der Übergabeposition des pharmazeutischen Produktes (T) an das Transportsystem ein Drehteller angeordet ist, auf welchem das Produkt (T) unmittelbar vor der kraft- und/oder formschlüssigen Aufnahme durch das Transportsystem zu liegen kommt.

13. Vorrichtung nach zumindest einem der Ansprüche 6 bis 12, **dadurch gekennzeichnet, dass** die Greiffläche zumindest einer der Greiferbacken (8) mit einer elastischen Schicht (9) und/oder einer für die bei der Durchstrahlungsmessung verwendeten Wellenlängen undurchlässigen Schicht (9) bedeckt ist.

14. Vorrichtung nach zumindest einem der Ansprüche 6 bis 13, **dadurch gekennzeichnet, dass** die Greiferbacken (8) nach innen hin über ein geklemmtes Produkt (T) ragende Auskragungen aufweisen, und dass die den Boden der Auskragung überragende Höhe der Greiferbacken (8) geringer ist als die Dicke der Produkte (T).

15. Vorrichtung nach einem der Ansprüche 1 bis 14, **dadurch gekennzeichnet, dass** das Transportsystem (2) zur Ausübung einer auf das Produkt (T) in Richtung auf die Auflage (6) hin wirkenden Anpresskraft ausgelegt ist.

16. Verfahren zur Bestimmung wenigstens eines physikalischen Parameters pharmazeutischer Produkte (T), wie beispielsweise Tabletten, Pillen, Oblongs oder dergleichen, wobei zumindest eine chemische oder physikalische Eigenschaft geprüft und das Produkt zwischen und/oder während und/oder nach zumindest einem Prüfvorgang durch ein automatisches Transportsystem (2) transportiert und gehandhabt wird, **dadurch gekennzeichnet, dass** der Transportweg des Produktes (T) und dessen räumliche Orientierung zumindest zum Teil unabhängig von der räumlichen apparativen Anordnung vorprogrammiert und vorzugsweise mittels eines Roboterarms (2) abgefahren wird.

17. Verfahren nach Anspruch 16, **dadurch gekennzeichnet, dass** zumindest eine Eigenschaft des Produkts (T) mittels einer Durchstrahlungs- oder Reflexionsmessung ermittelt wird.

18. Verfahren nach Anspruch 17, **dadurch gekennzeichnet, dass** zumindest für die Durchstrahlungs oder Reflexionsmessung das Produkt (T) in Bezug auf die Einrichtung zur Durchstrahlungsmessung ortsfest fixiert wird, vorzugsweise in Richtung einer Auflage (6) mit einer Andrückkraft beaufschlagt wird.

19. Verfahren nach Anspruch 17, **dadurch gekennzeichnet, dass** das Produkt während der Durchstrahlungs- oder Reflexionsmessung beweglich belassen wird, oder dass mehrere Messungen durchgeführt werden, vorzugsweise an unterschiedlichen Stellen des Produktes (T).

20. Verfahren nach zumindest einem der Ansprüche 16 bis 19, **dadurch gekennzeichnet, dass** das Produkt (T) während zumindest eines Teils des Transportweges mit dem Transportsystem (2), vorzugsweise einer Halteeinrichtung (3) des Transportsystems, kraftschlüssig und/oder formschlüssig gekoppelt gehalten wird.

21. Verfahren nach zumindest einem der Ansprüche 16 bis 20, **dadurch gekennzeichnet, dass** das Produkt (T) zumindest nach Abschluss aller Prüfungen mittels des Transportsystems (2) in einer Ablage (10) abgelegt oder an weitere Prüfsysteme, Ablagen oder Handhabungssysteme übergeben wird.
